# EUROPEAN PATENT APPLICATION

(11) **EP 3 530 191 A1**
(43) Date of publication of application: **28.08.2019**
(21) Application number: 18158858.3
(22) Date of filing: 27.02.2018
(51) Int. Cl.: A61B 8/00, A61B 5/00, A61B 8/08, A61B 90/00

(54) **ULTRASOUND HEAD COMBINING ULTRASOUND AND OPTICS**

(71) Applicant: Leica Instruments (Singapore) Pte. Ltd., 608924 Singapore (SG)
(72) Inventor: THEMELIS, George, 88131 Lindau (DE)
(74) Representative: Grünecker Patent- und Rechtsanwälte PartG mbB

(57) **Abstract**

The invention relates to an ultrasound head (2), to a medical observation device (1) and to a method for observing live biological tissue (4). In order to provide the surgeon with a means of quickly gaining information on sub-surface structure in the surgical area during the operation, the method comprises the steps of contacting the tissue (4) with an ultrasound head (2) and recording ultrasound data (36) and optical image data (34) through the ultrasound head (2). The ultrasound head (2) comprises a sensor side (6) for contacting the tissue (4) and an ultrasound receiver (10). The sensor side (6) is at least sectionwise optically transparent so that light may enter the ultrasound head (2).

## Description

The invention relates to an ultrasound head for the observation of live biological tissue.

During surgery, in particular during image-guided surgery, it is necessary to continuously match a pre-operative atlas of the operation area to the actual view. There are, however, situations in which the surgeon needs more detailed information about the actual state and location of sub-surface structures.

The present invention aims to provide a device and a method allowing a quick update of both surface and sub-surface information about the operation area for the surgeon during surgery.

This aim is achieved, according to the invention, by providing an ultrasound head for the observation of live biological tissue, the ultrasound head comprising a sensor side for contacting the tissue and an ultrasound receiver, wherein the sensor side is at least sectionwise optically transparent.

According to the inventive method for observing live biological tissue according to the invention, the steps of contacting the tissue with an ultrasound head and recording ultrasound data and optical image data through the ultrasound head are comprised.

With the device and method according to the invention, the surgeon may obtain sub-surface information from the ultrasound receiver and, at the same time, information about the optical appearance of the surface above the sub-surface structure.

The invention can be further improved by adding one or more of the following features, of which each has its own technical effect and can be combined independently with any of the remaining additional features.

For example, the ultrasound head may not only contain an ultrasound receiver but also an ultrasound transmitter, so that only a single head needs to be handled by the surgeon.

The sensor side has preferably at least one optically transparent section having transparency both with regard to light in the visible spectrum and in the IR or NIR spectrum. The at least one optically transparent section may also be transparent to ultrasound, at least in the range in which ultrasound waves are receives by the ultrasound receiver in operation.

According to another embodiment, an optical assembly may be provided, in particular be integrated into the ultrasound head, e.g. by being contained in the same housing of the ultrasound head as the ultrasound receiver and, if present, the ultrasound transmitter. The optical assembly may comprise at least one of an optical wage guide assembly and at least one camera. The optical waveguide assembly may comprise or consist of one or more optical fibers. At least one end of the optical waveguide assembly may face the optically transparent section. Thus, light entering the optically transparent section of the sensor side may enter the optical waveguide assembly and/or light emanating from the end of the optical fiber may also pass through the optically transparent section. Preferably, the end of the optical waveguide assembly is in contact with the optically transparent section. The optical waveguide may be arranged between the at least one optically transparent section and the camera.

In another embodiment, an ultrasound waveguide assembly may be provided which is arranged between the ultrasound receiver and the sensor side. Using an ultrasound waveguide allows the respective field of view of the optical and the ultrasound sensing systems to be optimized.

An optical axis of the optical waveguide assembly and/or the camera at the location of the optically transparent section is preferably parallel to the emission direction of the ultrasound transmitter. This allows information on the same area to be captured by both optical and ultrasound means. More preferably, the optical axis and the emission direction are coaxial, so that the region covered by the optical sensing and the ultrasound sensing is identical. For example, the optically transparent section may surround the section of the sensor side which is used for receiving ultrasound. Additionally or alternatively, an ultrasound waveguide may be provided which surrounds the optically transparent section and guide received ultrasound waves to the ultrasound receiver.

The camera may be a matrix camera or a line camera. It is preferable that the camera be sensitive in at least one of IR and NIR wavelengths, so that the fluorescent light of fluoropohres that have been injected into the tissue can be recorded.

A matrix camera may be used or configured as a multispectral or hyperspectral camera performing a spatial line scan, if a dispersive optical element is arranged between the camera and the optically transparent window. The dispersive optical element is preferably adapted to map different parts of the light spectrum onto different parts of the camera matrix.

The camera itself may be very small and be e.g. a camera as it is used in smartphones. An optical waveguide may be located between the optically transparent section and the camera, or the camera may directly face the optically transparent section.

The optical assembly may further comprise an illumination device adapted to generate illumination light emanating through said optically transparent section. The illumination device is preferably adapted to create illumination light which contains or consists of light in the visible light range and in the ultraviolet, IR and/or NIR range. The illumination device illuminates the live tissue under the at least one optically transparent section. The light from the illumination device may exit the ultrasound head through an optically transparent section which is separate from the optically transparent section through which light enters the optical assembly.

At least one of a beam-splitter device and filter arrangement may be located between the optically transparent section and at least one of the camera and the illumination device. The light received by the camera through the optically transparent section and the light emitted from the illumination device through the optically transparent section may be guided through the same waveguide, or optical fiber, respectively.

The ultrasound head may further comprise at least one standardized connector for connecting at least one of a fiber-optic connector and an image data connector. For example, for connecting a fiber-optic connector, different standards such as SC-DC, M12-FO, FSMA, LC, FC, amongst others, or an optical endoscope connector may be used. The fiber-optic connector may be used for data transfer and/or to guide the light from the optically transparent section to a remote camera, which may e.g. be integrated into a medical observation device, such as a microscope.

The image data connector may e.g. be an HDMI, USB, RGB, DVI and any other similar type of connector, including a virtual connector based on wireless connectivity.

In one embodiment, the optically transparent section may be flush with the sensor side. Being flush with the sensor side has the advantage that no dirt can accumulate between the optically transparent section and the remainder of the sensor side. In a particular embodiment, the optically transparent section may be a window set in the sensor side. The optically transparent section is preferably made from optical glass or crystal. Preferably, the sensor side is planar.

In another embodiment, the medical observation device, which may be a microscope or an endoscope, may comprise an ultrasound head as described above. The following further improvements of such a medical observation device also relate to the above-mentioned method.

Ultrasound image data of the tissue may be provided to the medical observation device, which ultrasound image data have been generated from ultrasound data from the ultrasound head. The medical observation device may, in one embodiment, comprise an image processor, which is adapted to compute the ultrasound image data from the ultrasound data from the ultrasound head. Alternatively, the ultrasound image data may be acquired from an ultrasound machine, which is connected to the medical observation device.

In another embodiment, optical image data of the tissue may be provided, e.g. by a camera recording the optical image data through the optically transparent section. Additionally or alternatively, optical image data may result from recording images by a microscope lens of the medical observation device, or by guiding light from the optically transparent section to an internal camera of the medical observation device, such as a camera which may also be used for acquiring images through the microscope lens.

The image processor is preferably adapted to identify at least one morphological structure in the optical image data, such as a vascular plexus structure. Further, the image processor is preferably adapted to identify the identified morphological structure also in the ultrasound image data. The image processor is preferably further adapted to elastically match the ultrasound image data to the optical image data based on the at least one identified morphological structure. An example of identification of such structures is given, for example, in Rouchdy, Y.; Cohen, L: "A geodesic voting method for the segmentation of tubular tree and centerlines", DOI:10.1109/ISBI.2011.5872566, pp. 979-983; Suri, J.; Laxminarayan, S.: "Angiography and Plaque Imaging: Advanced Segmentation Techniques", CRC Press, pp. 501-518; an example for elastic matching may be found in Gee, J.C..; Reivich, M.: Bajcsy, R.: "Elastically Deforming a Three-Dimensional Atlas to Match Anatomical Brain Images", IRCS Technical Reports Series, 192.

A microscope and/or a microscopy method configured for elastic matching of the ultrasound image data is an aspect of this invention which may be claimed independently of the ultrasound head and its design.

The ultrasound head may be connected to the microscope using an umbilical cord comprising at least one of a data and optical connector. The camera may be integrated in a handle of the ultrasound head or in the umbilical cord. Additionally or alternatively, an ultrasound machine may be mechanically combined with the microscope and in data transfer connection with the microscope so that ultrasound data may be displayed as ultrasound image data by at least one display device of the microscope. The at least one display device may comprise a three-dimensional display.

At least one of the ultrasound image data and the optical image data may be three-dimensional image data. Optical image data may e.g. be acquired by using z-stacking or SPIM and SCAPE technologies. Acquiring three-dimensional optical image data improves the accuracy of matching between three-dimensional ultrasound image data and optical image data.

The image processor may be adapted to combine the matched ultrasound image data and the optical image data into three-dimensional output image data. The output image data may be provided at an output interface of the medical observation device. In an embodiment, in which the medical observation device also comprises a display, such as a three-dimensional display, the output image data may be displayed on the at least one display, preferably in real time.

If a two-dimensional display is used, depth information may be displayed by assigning different false colors to different levels of depth in the output image data, i.e. distance from the visible surface.

In the following, embodiments of the invention as described exemplarily with reference to the accompanying drawings. In the drawings, identical reference numerals are used for elements which correspond to each other with respect to at least of function and design. It is clear from the above description of possible additional features, that the combination of features shown in the individual embodiments may be altered. For example, an additional feature may be added to an embodiment if, for a particular application, the technical effect of that additional feature is beneficial, and *vice versa:* a feature shown in an embodiment may be omitted if the technical effect of that feature is not necessary in a particular embodiment.

In the figures,
- Fig. 1: shows a medical observation device according to the invention;
- Figs. 2 to 6: show embodiments of an ultrasound head according to the invention.

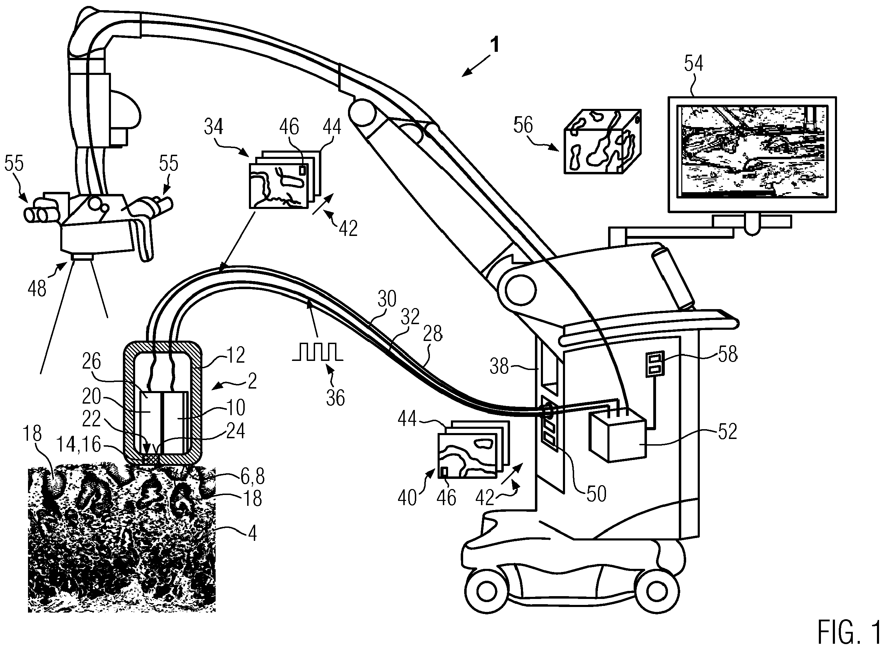

First, the design and function of a medical observation device 1, such as a microscope, in particular a surgical microscope, or endoscope, and of an ultrasound head 2 according to the invention are described with reference to Fig. 1.

Both the medical observation device 1 and the ultrasound head 2 are used to observe live biological tissue 4, e.g. organ tissue, such as brain tissue, vascular plexus, muscular or bone tissue. The ultrasound head 2 may in particular be used to obtain interoperative image data of the tissue 4.

The ultrasound head 2 is provided with a sensor side 6 which has a preferably planar surface 8. For ultrasound imaging, the sensor side 6, in particular the surface 8, is brought into contact with the tissue 4, as shown in Fig. 1.

The ultrasound head 2 comprises at least one ultrasound receiver 10, which is adapted to receive ultrasound waves through the sensor side 6. The ultrasound head 2 preferably also includes an ultrasound transmitter, which may be integrated into the ultrasound receiver 10 to form an ultrasound transceiver. Alternatively, the ultrasound transmitter and the ultrasound receiver 10 may be two separate units which are combined within a common housing 12 of the ultrasound head 2.

The sensor side 6 is at least sectionally optically transparent. The optically transparent section 14 may e.g. comprise a window 16. The optically transparent section 14 allows light to pass at least from the outside of the sensor side 6 into the ultrasound head 2 through the sensor side 6. Preferably, the optically transparent section 14 is transparent in both directions, i.e. also allows light to pass from the interior of the ultrasound head 2 through the sensor side 6 towards the tissue 4.

The optically transparent section 14 is flush with the sensor side 6, or the surface 8, respectively. Preferably, there is no gap or step between the optically transparent section 14 and the surrounding sensor side 6 or surface 8, respectively. The optically transparent section 14 is preferably made from optical glass and/or crystal. The transparency of the optically transparent section 14 preferably extends over the visible light range and may also cover ultraviolet and/or IR and NIR wavelengths. Transparency with regard to ultraviolet, IR and/or NIR wavelengths allows light to pass from the inside of the ultrasound head 2 to the outside that can be used to trigger fluorescence of fluorophores, such as indocyanine green, or that corresponds to fluorescence wavelengths. Transparency with regard to ultraviolet, IR and/or NIR wavelengths of the optically transparent section 14 towards the inside of the ultrasound head 2 allows optical information about the tissue 4 to be captured in the respective wavelengths, which may facilitate identification of morphological structures 18 in the tissue 4 and also enable fluorescence to be captured in the ultraviolet, IR and/or NIR wavelengths.

The ultrasound head 2 comprises an optical assembly 20 of which one end 22 faces the optically transparent section 14.

The optical assembly 20 may comprise an optical waveguide assembly which is adapted to capture, at its end 22, light entering the ultrasound head 2 through the optically transparent section 14 and guide it to a remote location, such as a fiber-optic connector or a camera. The optical waveguide assembly may comprise at least one optical fiber. To avoid losses, the optical waveguide assembly is preferably in contact with the optically transparent section 14 and its inside 24, respectively.

The optical assembly 20 may comprise a camera 26, which is shown exemplarily in Fig. 1. The camera 26 may, for example, be a camera as it is used in smartphones. The camera 26 may directly face the optically transparent section 14.

The ultrasound head 2 may be connected directly to the medical observation device 1 via an umbilical cord 28, which provides data connections 30, 32 between the medical observation device 1, on the one hand, and the optical assembly 20 and the ultrasound receiver 10, on the other. From the camera 26, optical image data 34 are transmitted from the camera 26 towards the medical observation device 1 via data connection 30. From the ultrasound receiver 10, ultrasound data 36 are transmitted to the medical observation device 1 via data connection 32. An ultrasound machine 38 may be integrated into the medical observation device 1 to generate ultrasound image data 40. At least one of the optical image data 34 and the ultrasound image data 40 may be three-dimensional. The optical image data 34 and the ultrasound image data 40 may consist of or comprise a time series 42 of individual frames 44. Each frame 44 may comprise a plurality of pixels 46. If the image data 34, 40 are three-dimensional, each frame 44 may itself be a three-dimensional data structure or atlas.

The camera 26 may be a multi- or hyperspectral camera and may perform line scans of the tissue 4. Additionally or alternatively, the camera 26 may work regularly and provide a matrix view of the tissue 4.

The medical observation device 1 preferably additionally comprises a lens 48, such as a microscope lens, which is arranged in front of a camera (not shown). This camera may also generate optical image data 34 that may be used in conjunction with or instead of the optical image data 34 which have been recorded through the optically transparent section 14. The camera behind the lens 48 may be connected instead of the camera 26 in the ultrasound head 2 for image capture and image analysis through the optically transparent section 14. Of course, the medical observation device 1 may allow connections with a plurality of cameras, so that the camera 26 and the camera behind the lens 48 may be connected in parallel. For this, the medical observation device 1 may comprise an input interface 50 which is adapted to connect a plurality of cameras to an image processor 52 of the medical observation device 1.

The image processor 52 may be adapted to identify the at least one morphological structure 18 in the tissue 4 in the optical image data 34 using e.g. one of the methods described in the articles mentioned in the introductory part of this specification.

The at least one morphological structure 18 identified in the optical image data 34 may then be used as a landmark to register the ultrasound image data 40 so that the geometric extent and location of morphological features in the ultrasound image data 40 corresponds to those in the optical image data 34. This is achieved in that the morphological structure 18, which has been identified in the optical image data 34 is next identified in the ultrasound image data 40. Then, the ultrasound image data 40 is further adapted to elastically match the optical image data 34 based on the at least identified morphological structure 18.

The medical observation device 1 may further comprise at least one display 54, e.g. displays 54 for the surgeon and the surgical assistant which are visible through eyepieces 55 and/or a display 54 which is visible to other assistants in the room. Preferably, one or more displays 54 are three-dimensional displays so that the three-dimensional information can be displayed more clearly if three-dimensional optical image data 34 or three-dimensional ultrasound image data 40 are used.

The image processor 52 may be adapted to combine the matched ultrasound image data 40 and the optical image data 34 into a preferably three-dimensional output image data 56 and to output the output image data 56 at an output interface 58 which provides connectors for connecting one or more displays 54.

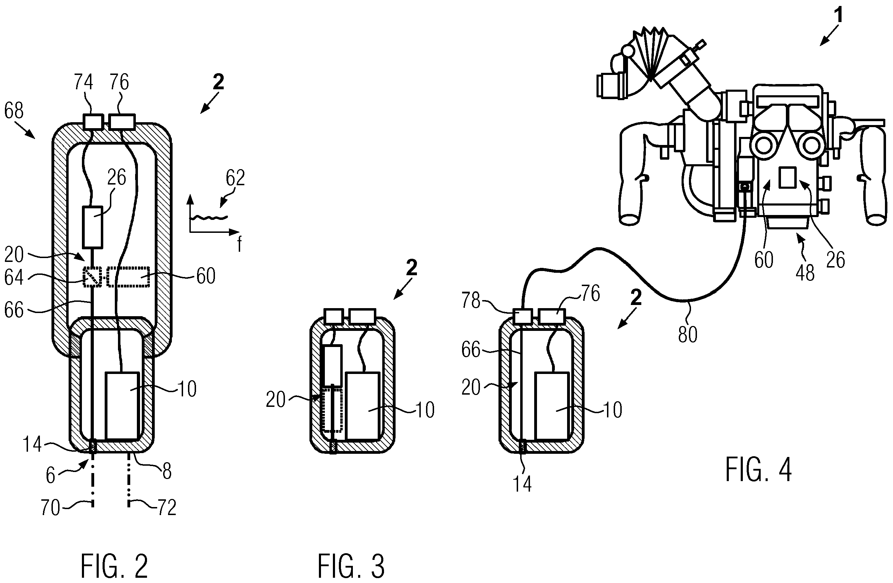

The ultrasound head 2 may have different configurations which are shown schematically in Figs. 2 to 6 and are described in the following. Only the differences with regard to the preceding embodiments are described.

In Fig. 2, the ultrasound head 2 comprises an illumination device 60, such as a light source comprising or consisting of LEDs. The illumination device 60 generates illumination light having an illumination spectrum 62 which preferably covers at least one of the visible light spectrum, the UV light spectrum, the IR spectrum and the NIR spectrum. The illumination device 60 is integrated into the ultrasound head 2 and e.g. contained within a housing which also contains the camera 26. The optical assembly 20 may comprise a mixer 64 which is adapted to couple the light generated by the illumination device 60 into an optical waveguide assembly 66, such as an optical fiber assembly containing one or more optical fibers.

A separate optical waveguide or optical fiber assembly may be provided for light entering the optically transparent section 14 and being guided to the camera 26, on the one hand, and for light exiting the illumination device 60 and directed outwards through the optically transparent section 14, on the other. Alternatively, a single waveguide assembly 66 may be used for both light entering the camera 26 and exiting the illumination device 60. In contrast to the ultrasound head 2 of Fig. 1, the camera 26 is not arranged directly behind the optically transparent section 14. Instead, the optical waveguide assembly 66 is arranged between the camera 26 and the optically transparent section 14.

The ultrasound head 2 may comprise a handle 68 in which at least one of the camera 26 and the illumination device 60 may be arranged. Additionally, the ultrasound receiver 10 and/or the ultrasound transmitter may also be arranged in the handle 68. In this case, an ultrasound waveguide (not shown) may be installed between the ultrasound receiver 10 and/or the ultrasound transmitter and the sensor side 6.

The optical axis 70 of the optical assembly 20 outside the ultrasound head 2 is preferably parallel to the sensor direction 72 of the ultrasound transmitter. The sensor direction 72 of the ultrasound receiver 10 may be determined by e.g. averaging the directional sensitivities in various directions, or by determining the direction in which the ultrasound sensor is most sensitive.

The ultrasound head 2 may be provided with a preferably standardized connector assembly 74 for establishing the data connection 30 so that the optical image data 34 may be transmitted to the medical observation device 1. The connector assembly 74 may comprise or consist of e.g. an HDMI, USB or any other type of connector. The ultrasound head 2 may further be provided with a connector assembly 76 which is also preferably standardized and used for transmission of ultrasound data 36.

In Fig. 3, an ultrasound head 2 is shown which differs from the ultrasound head 2 in Fig. 2 only in that no handle 68 is provided and in that the optical assembly 20 is thus contained in a much smaller space. The ultrasound head 2 is thus particularly suited to being inserted into surgical cavities which are only very small.

In the embodiment of Fig. 4, the optical assembly 20 does not comprise a camera 26 and/or an illumination device 60. Instead, an optical waveguide assembly 66 extends from the optically transparent section 14 to a preferably standardized optical connector assembly 78. Thus, any light passing through the optically transparent section 14 in or out of the ultrasound 2 is directly guided to the connector assembly 78. From there, a further optical waveguide may connect the ultrasound head 2 to at least one of a camera 26 and an illumination device 60. For example, an optical connection 80 may be used to connect the ultrasound head 2 to the medical observation device 1 and e.g. use the camera 26 which is installed in the medical observation device 1 for acquiring the optical image data 34 through the optically transparent section 14. At the same time, the illumination device 60, which is integrated into the medical observation device 1, may be used to send illumination light through the optically transparent section 14 onto the tissue 4 (Fig. 1). Thus, both the illumination device 60 and the camera 26, which are normally used to observe the tissue 4 through the lens 48, may be used. The ultrasound head 2 can therefore be simplified.

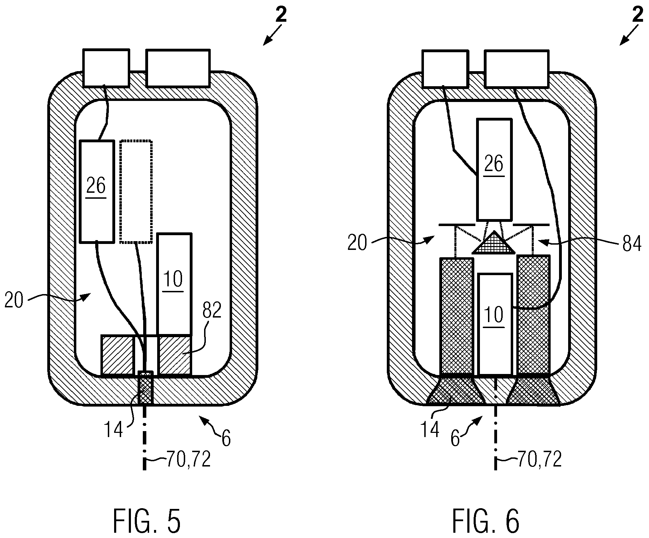

For some applications, it may be advantageous for the optical axis 70 and the sensor direction 72 to coincide, i.e. run coaxially. In Figs. 5 and 6, two embodiments of the ultrasound head 2 are shown in which this is realized.

According to the embodiment of Fig. 5, an ultrasound waveguide 82 surrounds the optically transparent section 14. The ultrasound waveguide 82 is arranged between the ultrasound receiver 10 and the sensor side 6, and is adapted to guide ultrasound at least from the sensor side 6 to the ultrasound receiver 10. The ultrasound waveguide 82 may have a ring shape. At its side facing the ultrasound receiver 10, the ultrasound waveguide 82 may be shaped to uniformly project the incoming ultrasound waves from around the optically transparent section 14 onto the ultrasound receiver 10. The optical assembly 20 may be in any configuration as described in the previous embodiments.

The general concept shown in Fig. 5 may also be used if the optically transparent section 14 surrounds the ultrasound receiver 10 or a ultrasound waveguide 82 connecting the sensor side 6 to the ultrasound receiver 10. This is shown exemplarily in Fig. 6.

The optical assembly 20 may comprise reflective elements 84 to guide the light from the annular optically transparent section 14 to the camera 26 or an optical waveguide assembly 66. Alternatively, the optical waveguide assembly 66 itself may, at its end facing the sensor side 6, transition to a ring shape.

Although, in the above embodiments, the sensor side 6 is shown with only a section 14 being optically transparent, it is also possible that the entire sensor side 6 is optically transparent, e.g. made from a glass plate if section 14 is, at the same time, transparent to ultrasound waves.

### REFERENCE NUMERALS

- 1: medical observation device
- 2: ultrasound head
- 4: live biological tissue
- 6: sensor side
- 8: surface of sensor side
- 10: ultrasound receiver
- 12: housing
- 14: optically transparent section
- 16: transparent window
- 18: morphological structure
- 20: optical assembly
- 22: end of optical assembly
- 24: inside of optically transparent section
- 26: camera
- 28: umbilical cord
- 30: data connection
- 32: data connection
- 34: optical image data
- 36: ultrasound data
- 38: ultrasound machine
- 40: ultrasound image data
- 42: time series of frames
- 44: frame
- 46: pixel
- 48: lens
- 50: input interface
- 52: image processor
- 54: display
- 55: eyepiece
- 56: output image data
- 58: output interface
- 60: illumination device
- 62: illumination spectrum
- 64: optical mixer
- 66: optical waveguide assembly
- 68: handle
- 70: optical axis
- 72: sensor direction
- 74: connector assembly for image transmission
- 76: connector assembly for ultrasound data transmission
- 78: optical connector assembly
- 80: optical connection
- 82: ultrasound waveguide
- 84: reflective element

## Claims

1. Ultrasound head (2) for the observation of live biological tissue (4), comprising a sensor side (6) for contacting the tissue (4) and an ultrasound receiver (10), wherein the sensor side (6) is at least sectionwise optically transparent.

2. Ultrasound head (2) according to claim 1, wherein an (22) of an optical assembly (20) is located adjacent to an optically transparent section (14) of the sensor side (6).

3. Ultrasound head (2) according to claim 2, wherein the optical assembly (20) comprises an optical waveguide assembly (66), of which one end (22) faces the optically transparent section (14).

4. Ultrasound head (2) according to claim 2 or 3, wherein the optical assembly (20) comprises at least one camera (26).

5. Ultrasound head (2) according to claims 3 and 4, wherein the optical waveguide assembly (66) is located between the optically transparent section (14) and the camera (26).

6. Ultrasound head (2) according to any one of claims 1 to 5, wherein the optical assembly (20) comprises an illumination device (60) adapted to create illumination light, the illumination light being directed through an optically transparent section (14) of the sensor side (6).

7. Ultrasound head (2) according to claim 6 and any one of claims 2 to 5, wherein the optical waveguide assembly (66) is located between the optically transparent section (14) and the illumination device (60).

8. Ultrasound head (2) according to claim 6 or 7, wherein the illumination light includes at least one of NIR and IR wavelengths.

9. Ultrasound head (2) according to any one of claims 2 to 8, wherein the optical assembly (20) comprises an optical waveguide assembly (66) which extends from the optically transparent section (14) to an optical connector assembly (78) accessible from outside the ultrasound head (2).

10. Ultrasound head (2) according to any one of claims 1 to 9, wherein the optically transparent sector (14) is flush with a surface (8) of the sensor side (6).

11. Medical observation device (1), such as a microscope or endoscope, comprising an ultrasound head (2) according to any one of claims 1 to 10, wherein ultrasound image data (40) of the tissue (4) are provided from ultrasound data (36) from the ultrasound head (2), wherein optical image data (34) of the tissue (4) are provided and wherein the medical observation device (1) comprises an image processor (52), which is adapted to identify at least one morphological structure (18) in the optical image data (34) to identify the at least one identified morphological structure (18) in the ultrasound image data (40) and to elastically match the ultrasound image data (40) to the optical image data (34) based on the at least one identified morphological structure (18).

12. Medical observation device (1) according to claim 11, wherein the image processor (52) is connected to a camera (26) of which the optical axis (70) is directed through the optically transparent section (14) of the ultrasound head (2).

13. Medical observation device (1) according to claim 11 or 12, wherein the image processor (52) is adapted to combine the matched ultrasound image data (40) and the optical image data (34) into three-dimensional output image data (56) and to output the three-dimensional output image data (56) to an output interface (58) for driving a three-dimensional display (54).

14. Microscope (1) according to any one of claims 11 to 13, wherein the microscope is provided with an input interface (50) adapted to be connected to at least one of an ultrasound receiver (10) and fiber optics.

15. Method for observing live biological tissue (4), comprising the steps of contacting the tissue (4) with an ultrasound head (2) and acquiring both ultrasound data (36) and optical image data (34) through the ultrasound head (2).
